# EUROPÄISCHE PATENTSCHRIFT

(11) **EP 3 291 863 B1**
(45) Veröffentlichungstag und Bekanntmachung des Hinweises auf die Patenterteilung: **13.03.2019**
(21) Anmeldenummer: 15720087.4
(22) Anmeldetag: 04.05.2015
(51) Int. Cl.: A61M 5/315, A61M 5/20

(54) **EINWEGINJEKTOR MIT ERHÖHTER AUSLÖSESICHERHEIT**
DISPOSABLE INJECTOR WITH INCREASED TRIGGERING RELIABILITY
INJECTEUR À USAGE UNIQUE À SÉCURITÉ DE DÉCLENCHEMENT ACCRUE

(43) Veröffentlichungstag der Anmeldung: 14.03.2018
(73) Patentinhaber: LTS Lohmann Therapie-Systeme AG, 56626 Andernach (DE)
(72) Erfinder: FORGHANI, Sara, 56182 Urbar (DE); WORTMANN, Uwe, 35037 Marburg (DE); HEUSER, Karsten, 53498 Bad Breisig (DE); SPILGIES, Heiko, 56068 Koblenz (DE)
(74) Vertreter: Thämer, Wolfgang
(86) Internationale Anmeldenummer: PCT/EP2015/059714
(87) Internationale Veröffentlichungsnummer: WO 2016/177393

(56) Entgegenhaltungen:
- WO-A1-01/72361
- WO-A1-2011/101383
- WO-A1-2012/105898
- WO-A1-2013/175144
- WO-A2-2014/154490

## Beschreibung

Die Erfindung betrifft einen Einweginjektor mit einem in einem Gehäuse gelagerten, mittels eines Federenergiespeichers belasteten und mittels einer verschiebbaren Auslösevorrichtung entsperrbaren Kolbenbetätigungsstempel, wobei der Kolbenbetätigungsstempel mittels eines im Gehäuse gelagerten Zugstabs abstützbar ist. Aus der DE 10 2008 063 519 A1 ist ein derartiger Einweginjektor bekannt. Das Auslösen kann durch Reibung und/oder Verkanten der Bauteile behindert werden.
Ein Einweginjektor gemäss dem Oberbegriff von Anspruch 1 ist aus dem Dokument WO 2014/154490 A2 bekannt. Der vorliegenden Erfindung liegt die Problemstellung zugrunde, die Auslösesicherheit eines Einweginjektors zu erhöhen.
Diese Problemstellung wird mit den Merkmalen des Hauptanspruchs gelöst. Dazu umfasst die Auslösevorrichtung einen relativ zum Gehäuse verschiebbaren Auslösering. Der Zugstab ist mittels einer Anlagefläche des Auslöserings direkt oder indirekt abstützbar. Außerdem schließt die Anlagefläche mit der Längsrichtung des Einweginjektors einen Winkel zwischen 10 Grad und 45 Grad ein, wobei der Scheitel des Winkels in der Auslöserichtung des Einweginjektors versetzt zum Auslösering liegt.
Weitere Einzelheiten der Erfindung ergeben sich aus den Unteransprüchen und den nachfolgenden Beschreibungen schematisch dargestellter Ausführungsbeispiele.
- Figur 1:: Einmalinjektor mit Verschlusskappe;
- Figur 2:: Explosionszeichnung des Einmalinjektors aus Figur 1;
- Figur 3:: Längsschnitt des Einmalinjektors aus Figur 1;
- Figur 4:: Längsschnitt normal zu Figur 3;
- Figur 5:: Querschnitt des Einmalinjektors oberhalb des Auslöserings;
- Figur 6:: Einmalinjektor nach dem Auslösen;
- Figur 7:: Einmalinjektor mit entleerter Zylinder-Kolben-Einheit;
- Figur 8:: Dimetrische Ansicht des Gehäuses;
- Figur 9:: Dimetrische Ansicht des Kolbenbetätigungsstempels;
- Figur 10:: Dimetrische Ansicht des Auslöserings;
- Figur 11:: Dimetrische Darstellung der Stützscheibe;
- Figur 12:: Dimetrische Darstellung des Stützstabs;
- Figur 13:: Detail der Auslösevorrichtung vor dem Auslösen;
- Figur 14:: Detail der Auslösevorrichtung nach dem Auslösen.

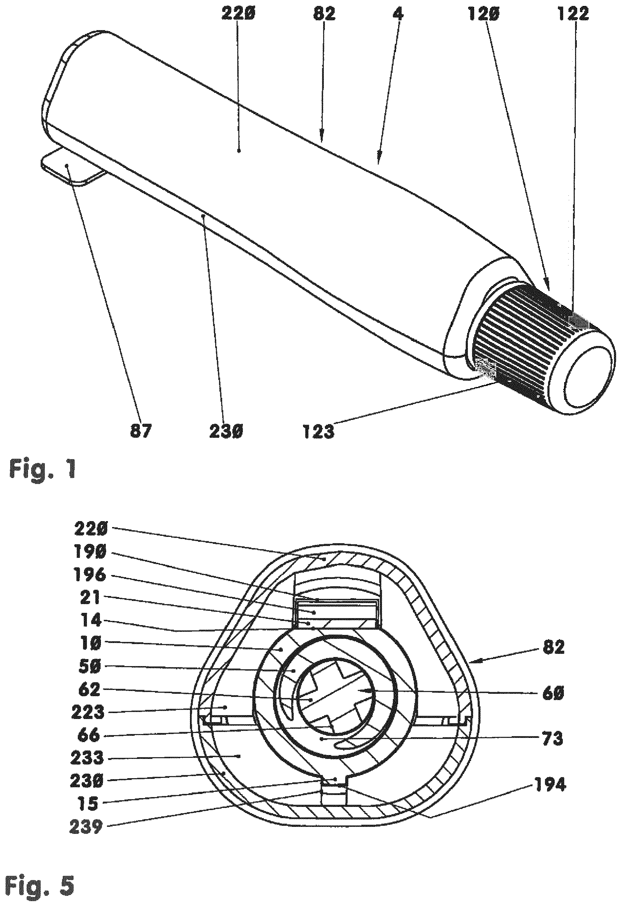

Die Figuren 1 - 14 zeigen einen Einmal- oder Einweginjektor (4). Derartige Injektoren (4) werden zum einmaligen Einbringen einer in einer Zylinder-Kolben-Einheit (100) gelagerten Injektionslösung (1) oder eines Lösemittels in die Haut eines Patienten eingesetzt.

Der Einweginjektor (4) umfasst ein Mantelgehäuse (82), in dem ein inneres Gehäuse (10) mit einem Kolbenbetätigungsstempel (60) und einer Schraubendruckfeder (50) als dauergeladener Federenergiespeicher (50) sowie eine Zylinder-Kolben-Einheit (100) angeordnet sind. Die Zylinder-Kolben-Einheit (100) ist mittels einer Schutzkappe (120) verschlossen. Das Auslösen des Einmalinjektors (4) wird mittels eines Sicherungsschiebers (87) verhindert. Dieser kann zur Entsicherung herausnehmbar sein oder er kann im Einmalinjektor (4) geführt verschiebbar sein. Das Gehäuse (10) und das Mantelgehäuse (82) sind beispielsweise aus Kunststoff hergestellt. Dies kann ein thermo- oder duroplastischer Werkstoff, z.B. POM, ABS, etc. sein.

Das Mantelgehäuse (82) besteht im Ausführungsbeispiel aus einer Oberschale (220) und einer Unterschale (230). Die beiden Schalen (220, 230) sind mittels Zapfenverbindungen (228, 238) miteinander verbunden und beispielsweise form-und/oder stoffschlüssig gesichert. Die Oberschale (220) und die Unterschale (230) können beispielsweise miteinander verklebt, verschweißt, etc. sein. Die Oberschale (220) und die Unterschale (230) können auch miteinander verrastet sein.

Das im Querschnitt polygonförmige Mantelgehäuse (82) hat im Ausführungsbeispiel einen zumindest annährend regelmäßigen, dreiecksähnlichen Querschnitt, vgl. Figur 5. Die Querschnittsfläche im hinteren, der Injektionsstelle abgewandten Bereich des Einweginjektors (4) beträgt 70 % der Querschnittsfläche im vorderen, der Injektionsstelle zugewandten Bereich des Einweginjektors (4). Der stetige Anstieg der Querschnittsfläche liegt, vom Bediener aus gesehen, im dritten Viertel der Länge des Einweginjektors (4).

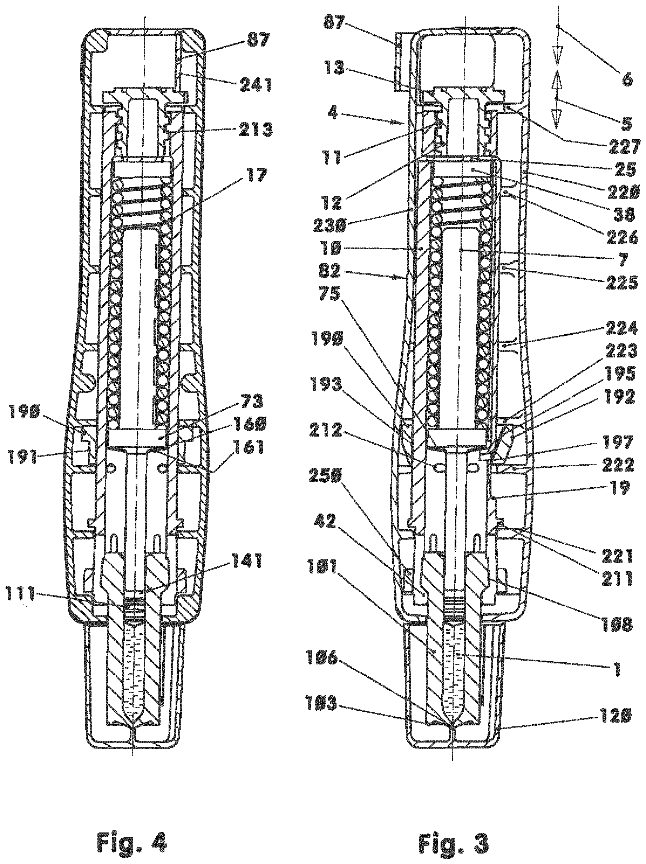

Der Einweginjektor (4) umfasst ein rohrförmig ausgebildetes Gehäuse (10), in dem ein Federenergiespeicher (50) und ein Kolbenbetätigungsstempel (60) angeordnet sind. Im vorderen, der Injektionsstelle zugewandten Bereich hat das Gehäuse (10) nach innen ragende Federhaken (42). In die Federhaken (42) ist in den Darstellungen der Figuren 3 und 4 die z.B. vorbefüllte Zylinder-Kolben-Einheit (100) eingesetzt und verrastet.

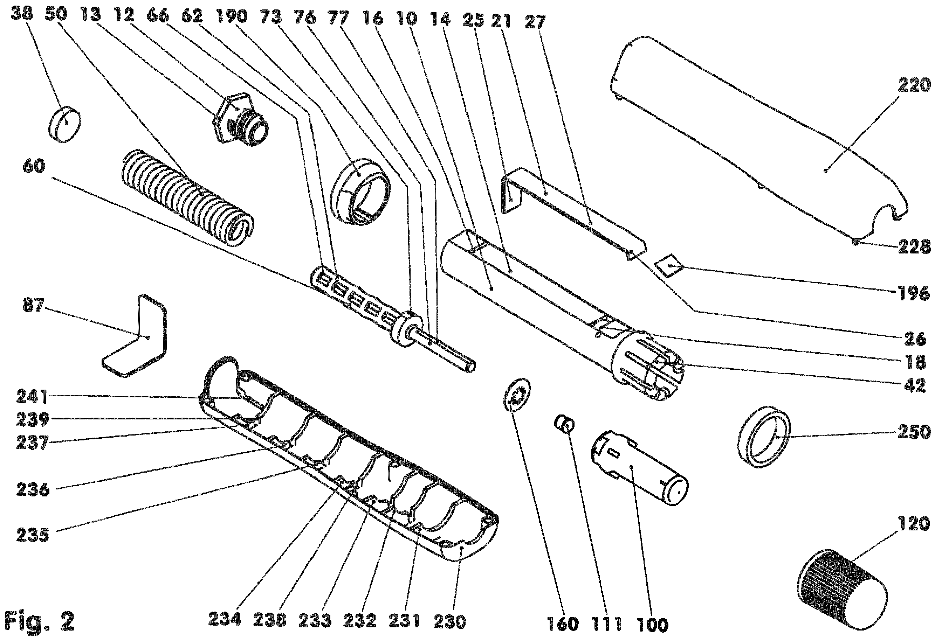

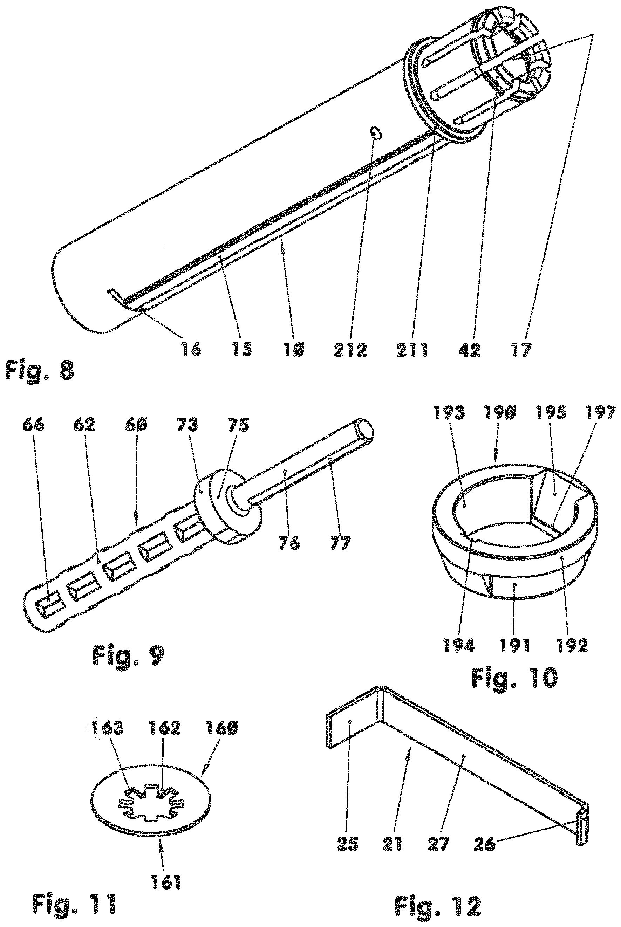

Das Gehäuse (10) hat eine weitgehend zylindrische Innenkontur. Im hinteren, der Injektionsstelle abgewandten Bereich ist ein Innengewinde (11) angeordnet. In diesem sitzt eine Abstützschraube (12) mit einem Sechskantabschnitt (13). Die Außenkontur des Gehäuses (10) hat eine zylinderförmige Grundform mit einer Abflachung (14), vgl. Figur 2. Im Bereich der Abflachung (14) liegt ein Stützstab (21) am Gehäuse (10) an. Der Stützstab (21) ragt mit einem Klemmschenkel (25) in einen oberen Schlitz (16) des Gehäuses (10). Ein Umgriffshaken (26) des Stützstabs (21) ragt durch eine untere Öffnung (18) mit rechteckiger Querschnittsfläche (18) in das Gehäuse (10). Gegenüber der Abflachung (14) weist das Gehäuse (10) eine in der Längsrichtung (5) des Einmalinjektors (4) orientierte Führungsrippe (15) auf, vgl. die Figuren 5 und 8.

Der Kolbenbetätigungsstempel (60) umfasst einen z.B. zylindrischen Führungszapfen (62), einen Stempelteller (73) und einen Kolbenschieber (76). Der Führungszapfen (62) trägt und führt den Federenergiespeicher (50), der hier als Schraubendruckfeder (50) ausgebildet ist. Das in den Figuren 3 und 4 obere Ende der Schraubendruckfeder (50) stützt sich über eine Scheibe (38) und den Klemmschenkel (25) des Stützstabs (21) an der Abstützschraube (12) ab. Der Führungszapfen (62) hat in radialer Richtung orientierte kreissegmentförmige Aussparungen (66). Der Einweginjektor (4) kann auch ohne die Stützscheibe (38) ausgeführt sein.

Der Stempelteller (73) des Kolbenbetätigungsstempels (60) ist scheibenförmig ausgebildet und normal zur Mittellängsachse (7) des Einweginjektors (4) ausgerichtet. Er hat eine der Schraubendruckfeder (50) abgewandte, konisch ausgebildete Bundfläche (75). Der Spitzenwinkel des gedachten Kegels der Bundfläche (75) beträgt z.B. 160 Grad.

An dieser Bundfläche (75) liegt eine Stützscheibe (160) an, vgl. Figur 11. Im Ausführungsbeispiel ist die Stützscheibe (160) eine Lochscheibe und hat um die zentrale Bohrung herum angeordnete Nuten (163) und Keile (162), mit denen sie formschlüssig auf dem Kolbenschieber (76) des Kolbenbetätigungsstempels (60) sitzt. Die Stützscheibe (160) ist in der Seitenansicht kegelstumpfförmig ausgebildet. Sie ist beispielsweise aus einem metallischen Werkstoff, z.B. einem austenitischen, korrosionsbeständigen Stahl, hergestellt. In der Darstellung der Figur 3 ist sie mittels des Umgriffshakens (26) des Stützstabs (21) in ihrer Position gesichert.

Der stangenartig ausgebildete Kolbenschieber (76) hat im Ausführungsbeispiel eine zumindest annähernd zylindrische Gestalt. Beispielsweise weist er in Längsrichtung des Einmalinjektors (4) ausgerichtete Schlüsselflächen (77) auf. Entlang dieser Schlüsselflächen (77) kann beim Eintauchen des Kolbenschiebers (76) in die Zylinder-Kolben-Einheit (100) Luft schneller entweichen.

Der Stützstab (21), vgl. die Figuren 2, 3 und 12, ist aus einem Blechstreifen mit z.B. konstanter rechteckiger Querschnittsfläche hergestellt. Die Breite des Stützstabes (21) normal zur Längsrichtung (5) des Einmalinjektors (4) ist im Ausführungsbeispiel acht Mal so groß wie seine Dicke. Der Werkstoff des Stützstabes (21) ist ein austenitischer Federstahl. Sein Elastizitätsmodul ist beispielsweise größer als 190.000 Newton pro Quadratmillimeter. Der Stützstab (21) umfasst einen Hauptschenkel (27), den Klemmschenkel (25) und den Umgriffshaken (26). In der Darstellung der Figur 3 liegt der Hauptschenkel (27) parallel zur Abflachung (14). Seine Länge beträgt beispielsweise dem Siebenfachen der Breite des Stützstabs (21). Das hintere, in der Figur 3 oben dargestellte Ende des Stützstabes (21) zeigt in Richtung des Innenraums (17) des Einweginjektors (4) und bildet den Klemmschenkel (25). Der z.B. durch ein Biegeumformverfahren abgewinkelte Klemmschenkel (25) schließt mit dem Hauptschenkel (27) einen Winkel von z.B. 92 Grad ein.

Das vordere, in der Figur 3 untere Ende des Stützstabs (21) zeigt ebenfalls in Richtung des Innenraums (17) und bildet den Umgriffshaken (26). Der Umgriffshaken (26) schließt mit dem Hauptschenkel (27) einen Winkel ein, der um den Neigungswinkel der Bundfläche (75) zu einer Normalenebene der Mittenlängsachse (7) des Einmalinjektors (4) größer ist als ein rechter Winkel. Die Länge des Umgriffshakens (26) beträgt beispielsweise 20 % der Länge des Klemmschenkels (25).

Die Zylinder-Kolben-Einheit (100) umfasst einen z.B. transparenten Zylinder (101) und einen im Zylinder (101) geführten Kolben (111). In den Darstellungen der Figuren 3, 4 und 6 sitzt der Kolben (111) in einer hinteren Position. Zwischen dem Kolben (111) und dem, ebenfalls im Zylinder (101) geführten Kolbenschieber (76) liegt ein Zwischenraum (141). Seine Länge beträgt im Ausführungsbeispiel zwei Millimeter. Diese Länge kann zwischen einem Millimeter und zehn Millimeter betragen.

Die in den Figuren 3, 4, 6 und 7 unten liegende Austrittsöffnung (106) der Zylinder-Kolben-Einheit (100) ist als kurze, zylindrische, düsenartige Bohrung (106) ausgebildet.

In den Darstellungen der Figuren 3, 4, 6 und 7 ist die Zylinder-Kolben-Einheit (100) in das Gehäuse (10) eingesetzt. Die Federhaken (42) hintergreifen den oberen Bund (108) der Zylinder-Kolben-Einheit (100). Auf den Federhaken (42) sitztunterhalb der Ebene des unteren Endes des Bundes (108) - ein Sicherungsring (250). Dieser Sicherungsring (250) hat im Ausführungsbeispiel eine kreisförmige Grundfläche. Sein Innendurchmesser ist beispielsweise größer als der Außendurchmesser des Gehäuses (10) im Bereich der unverformten Federhaken (42). Sein Außendurchmesser ist z.B. größer als der Innendurchmesser des Mantelgehäuses (82) und kleiner als der Außendurchmesser der Verschlusskappe (120).

Auf dem Gehäuse (10) sitzt ein Auslösering (190). Dieser ist als Einzelteil in der Figur 10 dargestellt. Seine Mantelfläche weist einen oberen zylindrischen Abschnitt (192) und einen unteren Abschnitt (191) auf. Der untere Abschnitt (191) ist bereichsweise zylindrisch und bereichsweise kegelstumpfförmig ausgebildet. Die in der Grundform zylinderförmige Innenwandung (193) hat auf der einen Seite eine in Längsrichtung (5) des Einweginjektors (4) orientierte Verdrehsicherungsnut (194). Auf der gegenüberliegenden Seite ist eine schräg ausgebildete Anlagefläche (195) ausgebildet. Diese Anlagefläche (195) ist beispielsweise in einem Winkel von 20 Grad zur Längsrichtung (5) des Einmalinjektors (4) ausgerichtet. Dieser Winkel, dessen Spitze in der Auslöserichtung (6) des Einweginjektors (4) versetzt zum Auslösering (190) liegt, kann zwischen 10 Grad und 45 Grad betragen. Die Anlagefläche (195) endet an einem unteren Absatz (197). Im Bereich dieses unteren Absatzes (197) ist die Innenwandung durch eine Sehne der Grundform begrenzt.

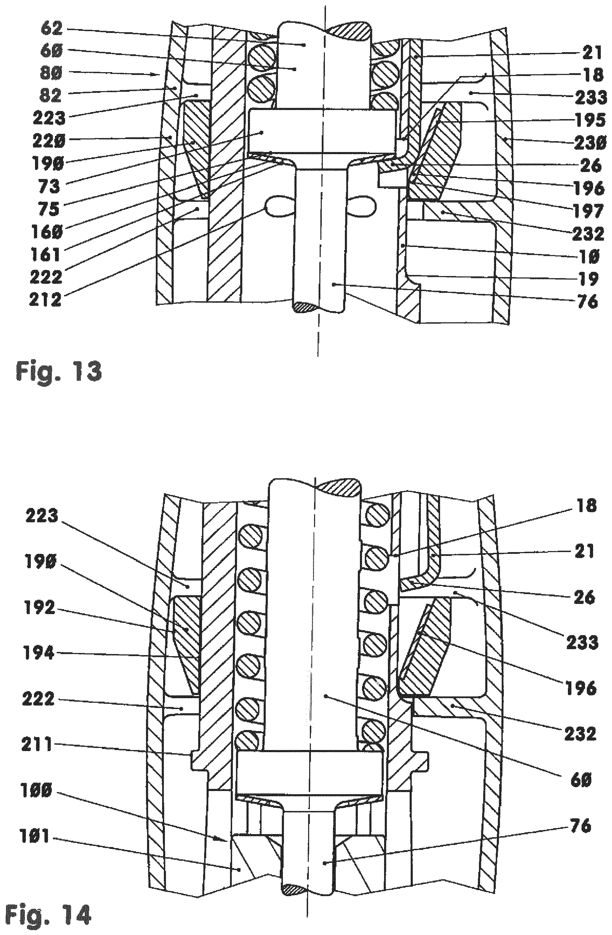

Auf der Anlagefläche (195) liegt im Ausführungsbeispiel ein als Gleitplatte (196) ausgebildetes Einschubblech (196). Der Einweginjektor (4) kann jedoch auch ohne das Einschubblech (196) ausgeführt sein. Das Einschubblech (196) besteht beispielsweise aus einem korrosionsfesten austenitischen Stahl. Der Elastizitätsmodul dieses Werkstoffs ist größer als 190.000 Newton pro Quadratmillimeter. Die Gleitplatte (196) steht auf dem Absatz (197) auf. Vor dem Auslösen des Einmalinjektors (4), vgl. Figur 3 und 13, ist der Stützstab (21) an der Gleitplatte (196) abgestützt. Er ist damit indirekt an der Anlagefläche (195) abgestützt. Die Haftreibungszahl dieser Werkstoffkombination ist beispielsweise geringer als 0,2. Auch bei längerer Lagerung verursacht der druckbelastete Stützstab (21) keine Verformungen der Gleitplatte (196). Damit ist auch nach längerer Lagerzeit ein sicheres Auslösen gewährleistet.

Die beiden Schalen (220, 230) der Auslösehülse (82) haben an ihrer Innenseite jeweils Verstärkungsrippen (221 - 227; 231 - 237). Diese Querrippen (221 -227; 231 - 237) sind normal zur Längsrichtung (5) des Einmalinjektors (4) orientiert. Hierbei weisen die hinteren Verstärkungsrippen (223 - 227; 232 - 237) jeweils zwei Teile auf, zwischen denen eine Nut (239) angeordnet ist. Die dem Bediener abgewandten vordersten Verstärkungsrippen (221, 222; 231) sind jeweils unterbrechungsfrei ausgeführt. Die Oberschale (220) und die Unterschale (230) sind mittels mehrerer Zapfenverbindungen (228, 238) miteinander verbunden. Hierbei weist im Ausführungsbeispiel die Oberschale (220) an der Trennfuge sechs Zapfen (228) auf, die in Zapfenlöcher (238) der Unterschale (230) eingreifen. Gegebenenfalls können die Zapfenverbindungen (228, 238) bei der Montage miteinander verrasten. Auch ein Verkleben der Unterschale (230) mit der Oberschale (220) ist denkbar.

Bei der Montage des Einmalinjektors (4) wird beispielsweise zunächst die Stützscheibe (160) auf den Kolbenschieber (76) des Kolbenbetätigungsstempels (60) aufgeschoben. Die Schraubenfeder (50) wird auf den Führungszapfen (62) des Kolbenbetätigungsstempels (60) aufgesetzt. Der Auslösering (190) wird, geführt an der Abflachung (14) und an der Führungsrippe (15), von hinten auf das Gehäuse (10) aufgeschoben, bis er unterhalb der rechteckigen Aussparung (18) liegt. Beispielsweise kann die Gleitplatte (196) bereits in den Auslösering (190) eingelegt und fixiert sein. Eine ringförmige Gehäuseaufweitung (19) verhindert das weitere Wandern oder Verschieben des Auslöserings (190) nach unten. Nach dem Einführen des Klemmschenkels (25) des Stützstabs (21) in den oberen Gehäuseschlitz (16) wird die Scheibe (38) von unten in das Gehäuse (10) eingelegt. Die Dicke der Scheibe (38) kann je nach erforderlicher Federvorspannung ausgewählt werden. Hiernach werden die vormontierten Teile (50, 60, 160) ebenfalls von unten in das Gehäuse (10) eingeführt, sodass die Schraubendruckfeder (50) an der Druckscheibe (38) anliegt und diese den Klemmschenkel (25) kontaktiert.

Nun kann die Abstützschraube (12) eingeschraubt werden, bis sie am Klemmschenkel (25) anliegt oder gegen diesen presst. Gegebenenfalls kann der Gewindegang (213) der Abstützschraube (12) und/oder des Gehäuses (10) eine Planverzahnung aufweisen, um ein unbeabsichtigtes Lösen der Abstützschraube (12) zu verhindern. Der Kolbenbetätigungsstempel (60) wird z.B. mittels eines Werkzeugs eingedrückt. Hierbei wird die Schraubendruckfeder (50) gespannt. Beispielsweise wird das Gehäuse (10) hierbei an einem Haltering (211) gehalten. Der Umgriffshaken (26) des Stützstabs (21) wird in die rechteckige Aussparung (18) eingeführt und an die Unterseite (161) der Stützscheibe (160) angelegt. Der Auslösering (190) wird nach oben gezogen, bis er am Stützstab (21) anliegt. Der Stützstab (21) stützt sich nun an der Gleitplatte (196) ab, vgl. Figur 13. Zur Sicherung der Montageposition kann ein z.B. u-förmiger Bügel in Montageöffnungen (212) des Gehäuses (10) eingeführt werden. Dieser Bügel sichert die Lage des Auslöserings (190) nach dem Abnehmen der Spannvorrichtung des Federenergiespeichers (50). Diese Vormontagegruppe kann nun z.B. an einen weiteren Arbeitsplatz gefördert werden. Es besteht keine Gefahr eines unbeabsichtigten Auslösens.

Im unteren Bereich des Einmalinjektors (4) wird der Sicherungsring (250) auf die Federhaken (42) aufgeschoben, bis er beispielsweise am Haltering (211) anliegt. Nun kann die z.B. vorbefüllte Zylinder-Kolben-Einheit (100) in das Gehäuse (10) eingesetzt werden und in diesem verrastet werden. Der Sicherungsring (250) wird nach vorne gezogen und sichert so die Lage der Zylinder-Kolben-Einheit (100).

Die Vormontagegruppe mit dem Gehäuse (10) und der Zylinder-Kolben-Einheit (100) kann nun weitergefördert oder behandelt werden.

Bei der Endmontage wird diese Vormontagegruppe beispielsweise in die Unterschale (230) eingelegt. Hierbei wird die Rippe (15) des Gehäuses (10) in der Längsnut (239) der Unterschale (230) zentriert. Der Auslösering (190) liegt zwischen den zweiten Querrippen (222, 232) und den dritten Querrippen (223, 233). Der Kopf der Abstützschraube (12) ragt über die hinterste Querrippe (227; 237) heraus. Die Verschlusskappe (120) sitzt außerhalb des Mantelgehäuses (82). Das Sicherungselement (87) wird in den Schlitz (241) der Unterschale (230) eingeführt und z.B. zwischen der Abstützschraube (12) und dem Gehäuse (82) geklemmt. Die Abstützschraube (12) kann gegen weiteres Verdrehen z.B. formschlüssig gesichert werden. Gegebenenfalls kann eine zusätzliche Druckfeder zwischen der Abstützschraube (12) und der Hülse (82) den Widerstand gegen versehentliches Auslösen erhöhen. Diese Feder bestimmt auch den Widerstand des Einweginjektors (4) beim Auslösen. Der u-förmige Bügel kann entfernt werden.

Zum Abschluss der Montage wird die Oberschale (220) auf die Unterschale (230) aufgesetzt und z.B. durch Verkleben, Verrasten, etc. gesichert. Nun kann ein zusätzlicher Originalitätsverschluss, z.B. eine Banderole, über das Mantelgehäuse (82) und die Verschlusskappe (120) angebracht werden. An ihrer Umfangsfläche (122) weist sie eine Riffelung (123) auf, um ein Abrutschen der Finger zu vermeiden.

Es ist auch denkbar, die Montage in einer anderen als in der beschriebenen Reihenfolge durchzuführen.

Der fertig montierte Einweginjektor (4) kann nun verpackt und vertrieben werden. Wird er z.B. nach dem Auspacken auf einem Tisch abgelegt, besteht aufgrund der Gehäusegeometrie keine Gefahr des Wegrollens.

Vor dem Einsatz des Einmalinjektors (4) wird zunächst der Originalitätsverschluss entfernt. Nach dem Abnehmen der Verschlusskappe (120) kann das Sicherungselement (87) herausgezogen werden. Der Einmalinjektor (4) ist nun einsatzbereit und wird beispielsweise auf die Haut des Patienten aufgesetzt. Beispielsweise haftet die Stirnfläche (103) der Zylinder-Kolben-Einheit (100) an der Haut des Patienten an. Auch in diesem Zustand verhindert die Selbsthemmung zwischen dem Stützstab (21) und dem Auslösering (190) ein unbeabsichtigtes Selbstauslösen des Einweginjektors (4), vgl. die Figur 13.

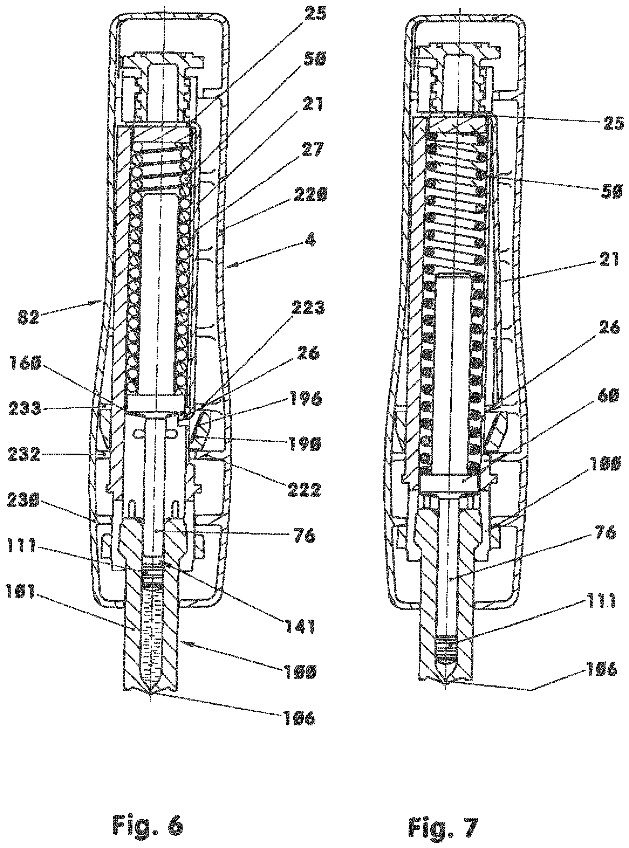

Zum Auslösen des Einmalinjektors (4) wird das Mantelgehäuse (82), das eine Auslösehülse (82) bildet, in der Auslöserichtung (6) nach vorne, also in Richtung der Haut des Patienten verschoben. Die Auslösehülse (80) verschiebt hierbei den Auslösering (190) relativ zum Gehäuse (10) in den Schnittdarstellungen der Figuren 3, 4, 6 und 7 nach unten. In der Figur 6 ist der nichtstatische Zustand unmittelbar nach dem Auslösen dargestellt. Der Federenergiespeicher (50) drückt den Stempelteller (73) nach vorne. Hierbei verdrängt die Druckscheibe (160) den Umgriffshaken (26) des Stützstabs (21). Der Stützstab (21) gleitet entlang der Gleitplatte (196) nach außen und gibt damit den Kolbenbetätigungsstempel (60) vollständig frei. Hierbei kann der Stützstab (21) gegebenenfalls auf eine dämmende Gummischicht auftreffen. Der Kolbenbetätigungsstempel (60) schnellt, belastet von dem sich entspannenden Federenergiespeicher (50), nach vorne bzw. unten. Der Kolbenschieber (76) schlägt auf den Kolben (111) und schiebt diesen nach vorne. Hierbei wird die Luft aus dem Zwischenraum (141) entlang der Schlüsselflächen (77) verdrängt. Die im Zylinder (101) gelagerte Injektionslösung (1) wird durch die Austrittsöffnung (106) und die Hornhaut des Patienten hindurch in den Körper des Patienten verdrängt.

Die Figuren 7 und 14 zeigen den Einmalinjektor (4) nach dem Auslösen. Der Auslösering (190) ist relativ zum Gehäuse (10) nach unten verschoben. Der Stützstab (21) ist nach außen verdrängt. Hierbei blockiert er z.B. das Wiedereinschieben der Auslösehülse (82) mit dem Auslösering (190). Der Federenergiespeicher (50) ist entspannt. Der Kolbenbetätigungsstempel (60) liegt in seiner vorderen Endlage. Die Zylinder-Kolben-Einheit (100) ist entleert.

Selbstverständlich ist es auch denkbar, die verschiedenen genannten Ausführungsformen miteinander zu kombinieren.

### Bezugszeichenliste:

- 1: Injektionslösung, Wasser für Injektionszwecke
- 4: Einmalinjektor, Einweginjektor
- 5: Längsrichtung
- 6: Auslöserichtung
- 7: Mittellängsachse

- 10: Gehäuse
- 11: Innengewinde
- 12: Abstützschraube
- 13: Sechskantabschnitt
- 14: Abflachung
- 15: Führungsrippe
- 16: Gehäuseöffnung, schlitzförmig
- 17: Innenraum
- 18: Gehäuseöffnung mit rechteckigem Querschnitt
- 19: Gehäuseaufweitung

- 21: Stützstab, Sperrstab

- 25: Klemmschenkel
- 26: Umgriffshaken
- 27: Hauptschenkel

- 38: Scheibe
- 42: Federhaken

- 50: Federenergiespeicher, Schraubendruckfeder, Feder

- 60: Kolbenbetätigungsstempel

- 62: Führungszapfen
- 66: Aussparungen

- 73: Stempelteller
- 75: Bundfläche
- 76: Kolbenschieber
- 77: Schlüsselflächen

- 80: Auslöseeinheit
- 82: Auslöseelement, Auslösehülse

- 87: Sicherungsschieber, Sicherungselement

- 100: Zylinder-Kolben-Einheit
- 101: Zylinder
- 103: Stirnfläche
- 106: Bohrung, Austrittsöffnung

- 108: Bund

- 111: Kolben

- 120: Verschlusskappe

- 122: Umfangsfläche
- 123: Riffelung

- 141: Zwischenraum

- 160: Stützscheibe, Druckscheibe
- 161: Unterseite
- 162: Keile
- 163: Nuten

- 190: Auslösering
- 191: unterer Bereich
- 192: zylinderförmiger Bereich
- 193: Innenwandung
- 194: Verdrehsicherungsnut
- 195: Anlagefläche
- 196: Einschubblech, Gleitplatte
- 197: Absatz

- 211: Haltering
- 212: Montageöffnungen
- 213: Gewindegang

- 220: Oberschale
- 221, 222: Verstärkungsrippen, Querrippen, einteilig
- 223 - 227: Verstärkungsrippen, Querrippen, zweiteilig
- 228: Zapfen

- 230: Unterschale
- 231: Verstärkungsrippe, Querrippe, einteilig
- 232 - 237: Verstärkungsrippe, Querrippe, zweiteilig
- 238: Zapfenlöcher
- 239: Längsnut

- 241: Schlitz

- 250: Sicherungsring

## Patentansprüche

1. Einweginjektor (4) mit einem in einem Gehäuse (10) gelagerten, mittels eines Federenergiespeichers (50) belasteten und mittels einer verschiebbaren Auslösevorrichtung (80) entsperrbaren Kolbenbetätigungsstempel (60), wobei der Kolbenbetätigungsstempel (60) mittels eines im Gehäuse (10) gelagerten Zugstabs (21) abstützbar ist,
**dadurch gekennzeichnet,**
- **dass** die Auslösevorrichtung (80) einen relativ zum Gehäuse (10) verschiebbaren Auslösering (190) umfasst,
- **dass** der Zugstab (21) mittels einer Anlagefläche (195) des Auslöserings (190) direkt oder indirekt abstützbar ist und
- **dass** die Anlagefläche (195) mit der Längsrichtung (5) des Einweginjektors (4) einen Winkel zwischen 10 Grad und 45 Grad einschließt, wobei der Scheitel des Winkels in der Auslöserichtung (6) des Einweginjektors (4) versetzt zum Auslösering (190) liegt.

2. Einweginjektor (4) nach Anspruch 1,
**dadurch gekennzeichnet,**
**dass** der Auslösering (190) in einer Auslösehülse (82) der Auslösevorrichtung (80) gelagert ist.

3. Einweginjektor (4) nach Anspruch 1,
**dadurch gekennzeichnet,**
**dass** der Auslösering (190) und das Gehäuse (10) verdrehsicher zueinander ausgebildet sind.

4. Einweginjektor (4) nach Anspruch 1,
**dadurch gekennzeichnet,**
**dass** die Anlagefläche (195) eine Gleitplatte (196) trägt.

5. Einweginjektor (4) nach Anspruch 1,
**dadurch gekennzeichnet,**
**dass** der Zugstab (21) einstreifig ausgebildet ist.

6. Einweginjektor (4) nach Anspruch 1,
**dadurch gekennzeichnet,**
**dass** der Zugstab (21) mittels eines Klemmschenkels (25) im Gehäuse (10) gelagert ist.

7. Einweginjektor (4) nach Anspruch 6,
**dadurch gekennzeichnet,**
**dass** der Klemmschenkel (25) im Gehäuse (10) mittels einer im Gehäuse (10) befestigten Abstützschraube (12) fixiert ist.

8. Einweginjektor (4) nach Anspruch 1,
**dadurch gekennzeichnet,**
**dass** die Relativbewegung zwischen der Auslösevorrichtung (80) und dem Gehäuse (10) mittels eines lösbaren oder entriegelbaren Sicherungselements (87) blockierbar ist.

## Claims

1. A single-use injector (4) having a piston actuation ram (60) which is mounted in a housing (10), is loaded by means of a spring energy store (50), and can be released by means of a displaceable triggering device (80), wherein the piston actuation ram (60) can be supported by means of a tension bar (21) which is mounted in the housing (10),
**characterised in that**
- the triggering device (80) comprises a triggering ring (190) displaceable relative to the housing (10),
- the tension bar (21) can be supported directly or indirectly by means of a bearing face (195) of the triggering ring (190), and
- the bearing face (195) encloses an angle between 10 degrees and 45 degrees with the longitudinal direction (5) of the single-use injector (4), wherein the apex of the angle is offset in the triggering direction (6) of the single-use injector (4) relative to the triggering ring (190).

2. The single-use injector (4) according to claim 1, **characterised in that** the triggering ring (190) is mounted in a triggering sleeve (82) of the triggering device (80).

3. The single-use injector (4) according to claim 1, **characterised in that** the triggering ring (190) and the housing (10) are prevented from rotating relative to one another.

4. The single-use injector (4) according to claim 1, **characterised in that** the bearing face (195) carries a sliding plate (196).

5. The single-use injector (4) according to claim 1, **characterised in that** the tension bar (21) is formed as a single strip.

6. The single-use injector (4) according to claim 1, **characterised in that** the tension bar (21) is mounted in the housing (10) by means of a clamping limb (25).

7. The single-use injector (4) according to claim 6, **characterised in that** the clamping limb (25) is fixed in the housing (10) by means of a support screw (12) fastened in the housing (10).

8. The single-use injector (4) according to claim 1, **characterised in that** the relative movement between the triggering device (80) and the housing (10) can be blocked by means of a releasable or unlockable securing element (87).

## Revendications

1. Injecteur à usage unique (4), comprenant un poinçon d'actionnement de piston (60) logé dans un boîtier (10) qui est sollicité par un accumulateur d'énergie à ressort (50) et qui peut être débloqué à l'aide d'un dispositif de déclenchement coulissant (80), le poinçon d'actionnement du piston (60) pouvant être soutenu à l'aide d'une tige de traction (21) montée dans le boîtier (10),
**caractérisé en ce**
- **que** le dispositif de déclenchement (80) comprend une bague de déclenchement (190) déplaçable par rapport au boîtier (10),
- **que** la tige de traction (21) peut être soutenue directement ou indirectement à l'aide d'une surface d'appui (195) de la bague de déclenchement (190) et
- **que** la surface d'appui (195) et la direction longitudinale (5) de l'injecteur à usage unique (4) renferment un angle entre 10 degrés et 45 degrés, le sommet de l'angle se trouvant décalée dans la direction de déclenchement (6) de l'injecteur à usage unique (4) par rapport à la bague de déclenchement (190).

2. Injecteur à usage unique (4) selon la revendication 1, **caractérisé en ce**
**que** la bague de déclenchement (190) est logée dans un manchon de déclenchement (82) du dispositif de déclenchement (80).

3. Injecteur à usage unique (4) selon la revendication 1, **caractérisé en ce**
**que** la bague de déclenchement (190) et le boîtier (10) sont bloqués en rotation l'une par rapport à l'autre.

4. Injecteur à usage unique (4) selon la revendication 1, **caractérisé en ce**
**que** la surface d'appui (195) porte une plaque coulissante (196).

5. Injecteur à usage unique (4) selon la revendication 1, **caractérisé en ce**
**que** la tige de traction (21) a une forme plate et allongée.

6. Injecteur à usage unique (4) selon la revendication 1, **caractérisé en ce**
**que** la tige de traction (21) est montée dans le boîtier au moyen d'une branche de serrage (25).

7. Injecteur à usage unique (4) selon la revendication 1, **caractérisé en ce**
**que** la branche de serrage est bloquée dans le boîtier (10) au moyen d'une vis d'appui (12) fixée dans le boîtier (10).

8. Injecteur à usage unique (4) selon la revendication 1, **caractérisé en ce**
**que** le mouvement relatif entre le dispositif de déclenchement (80) et le boîtier (10) peut être bloqué au moyen d'un élément de sécurité amovible ou déverrouillable (87).
